(19) Europäisches Patentamt
European Patent Office
Office européen des brevets

(11) **EP 2 402 075 A1**

(12) **EUROPÄISCHE PATENTANMELDUNG**

(43) Veröffentlichungstag:
**04.01.2012 Patentblatt 2012/01**

(51) Int Cl.:
**B01J 13/04** (2006.01)  **A61K 9/50** (2006.01)

(21) Anmeldenummer: **10167570.0**

(22) Anmeldetag: **28.06.2010**

(84) Benannte Vertragsstaaten:
**AL AT BE BG CH CY CZ DE DK EE ES FI FR GB GR HR HU IE IS IT LI LT LU LV MC MK MT NL NO PL PT RO SE SI SK SM TR**
Benannte Erstreckungsstaaten:
**BA ME RS**

(71) Anmelder: **Bühler AG**
**9240 Uzwil (CH)**

(72) Erfinder: **Bohm, Arturo**
**9242 Oberuzwil (CH)**

(74) Vertreter: **Hepp Wenger Ryffel AG**
**Friedtalweg 5**
**9500 Wil (CH)**

(54) **Verfahren und Vorrichtung zur Herstellung von Vesikeln**

(57)     Die Erfindung betrifft ein Verfahren zur Herstellung von Vesikeln mit den Schritten: a) Bereitstellen eines Fluides (1), welches zumindest eine amphiphile Substanz enthält; b) Erzeugung einer Strömung des Fluides (1) in mindestens einem Strömungsraum (19), wobei die Strömung wenigstens in einem Teilbereich des Strömungsraumes (19) zumindest einen Dehnströmungsanteil enthält, der die Bildung von Vesikeln aus dem Fluid (1) bewirkt. Des Weiteren ist eine Vorrichtung zur Herstellung von Vesikeln offenbart, die mindestens ein Paar von drehbaren Walzen (21,21') mit jeweils einer im Wesentlichen zylindermantelförmigen Walzfläche (22,22') enthält, wobei zwischen den Walzen (21,21) ein Strömungsraum (19) für ein Fluid (1) gebildet ist. Mindestens eine der Walzen (21) weist einen Hohlraum (23) auf. Die Walzfläche (22) dieser Walze (21) weist mindestens eine Einströmöffnung auf, durch welche ein Fluid (1) vom Strömungsraum (19) in den Hohlraum (23) strömen kann.

Figur 4

**Beschreibung**

Die vorliegende Erfindung betrifft Verfahren zur Herstellung von Vesikeln und Vorrichtungen zur Herstellung von Vesikeln.

Vesikel umfassen eine geschlossene Anordnung einer Doppelschicht amphiphiler Moleküle und bilden u. a. selbstorganisierende und/oder selbstaggregierende Kügelchen oder Kapseln. Vesikel sind meist sphärisch geformte Gebilde, die im Aussenbereich eine einzige oder mehrere Doppelschichten enthalten und im Inneren eine beispielsweise wässrige Phase einschliessen. Die Doppelschicht kann beispielsweise Lipide enthalten, wie etwa Phospholipide. In ihrem Inneren können insbesondere hydrophile Wirkstoffmoleküle eingelagert sein, und in ihrem Randbereich können insbesondere hydrophobe Moleküle vorhanden sein können.

Derartige Vesikel können auf verschiedene Arten hergestellt werden. Ein mögliches Verfahren ist beispielsweise in der europäischen Patentanmeldung EP 10159108.9 offenbart, welche zum Zeitpunkt der Einreichung der vorliegenden Anmeldung noch nicht veröffentlicht war. Bei diesem Verfahren wird ein erstes Fluid, welches eine amphiphile Substanz enthält, durch eine poröse Membran hindurchgeführt. Auf der Rückseite der Membran bilden sich Tropfen, die von einem zweiten Fluid abgeschert werden, so dass sich Vesikel bilden. Einige erste Fluide können die Membran aufgrund ihrer Viskosität oder anderer Eigenschaften verstopfen oder sogar zerstören. Daher können mit diesem bekannten Verfahren nur bestimmte Fluide mit bestimmten Zusammensetzungen, Konzentrationen und Viskositäten eingesetzt werden, was den Anwendungsbereich de Verfahrens einschränkt.

Weiterhin wird in EP 664 116 ein Verfahren zur Herstellung von Liposomen beschrieben, bei dem eine geeignete Zusammensetzung in einer Rührwerks-Kugelmühle gemahlen wird. Bei diesem Verfahren werden die Liposomen durch die in der Kugelmühle auftretenden Scherkräfte erzeugt. Auch in WO 89/00846 wird die Herstellung von Liposomen durch Scheren beschrieben. Die für die Erzeugung von Scherströmungen einsetzbaren Vorrichtungen sind jedoch baulich aufwendig. Zudem muss das Fluid für eine relativ lange Zeit der Scherströmung ausgesetzt werden, damit sich Vesikel bilden. Folglich muss auch die Vorrichtung eine gewisse Mindestgrösse haben, damit überhaupt Vesikel erzeugt werden können.

Es ist daher eine Aufgabe der vorliegenden Erfindung, ein Verfahren zur Herstellung von Vesikeln bereitzustellen, welches für eine grössere Vielfalt von Fluiden anwendbar ist und welches mittels baulich einfacher und kleinerer Vorrichtungen durchführbar ist.

Diese und weitere Aufgaben werden durch ein Verfahren zur Herstellung von Vesikeln und eine Vorrichtung zur Herstellung von Vesikeln gemäss den unabhängigen Patentansprüchen gelöst. Das Verfahren umfasst die folgenden Schritte:

a) Bereitstellen eines Fluides, welches zumindest eine amphiphile Substanz enthält;

b) Erzeugung einer Strömung des Fluides in mindestens einem Strömungsraum, wobei die Strömung wenigstens in einem Teilbereich des Strömungsraumes zumindest einen Dehnströmungsanteil enthält, der die Bildung von Vesikeln aus dem Fluid bewirkt.

Gegenüber den aus dem Stand der Technik bekannten Verfahren werden die Vesikel also unter anderem durch einen Dehnströmungsanteil erzeugt. Ausser dem Dehnströmungsanteil kann die Strömung optional zusätzlich einen Scherströmungsanteil und/oder Turbulenzen aufweisen.

Es wird derzeit vermutet (ohne dass die Erfindung hierauf zu beschränken ist), dass der Dehnströmungsanteil zu einer Streckung im Fluid enthaltener lamellarer Strukturen führt, diese gestreckten lamellaren Strukturen aufgrund der Dehnkräfte auseinander gerissen werden und sich anschliessend zu Vesikeln umformen. Dieser Effekt ist grösser als bei reinen Scherströmungen und umso ausgeprägter, je grösser der Dehnströmungsanteil im Fluid ist.

Bevorzugt beträgt das Volumen des Teilbereiches des Strömungsraumes, in dem der Dehnströmungsanteil vorliegt, mindestens 25 %, bevorzugt in mindestens 50 %, besonders bevorzugt in mindestens 75 % des Volumens des gesamten Strömungsraumes.

Bevorzugt liegt das in Schritt a) bereitgestellte Fluid in einer geordneten Phase vor. Unter geordneten Phasen werden dabei insbesondere die Phasen mit der Nomenklatur nach Luzzati verstanden, so wie sie im Abschnitt II.6 in D. Marsh, Handbook of Lipid Bilayers dargestellt sind:

- L    = eindimensional lamellar ("one-dimensional, lamellar") ;

- P    = zweidimensional schief oder zentriert ("two-dimensional oblique or centered (rippled)");

- H    = zweidimensional hexagonal ("two-dimensional hexagonal") ;

- $H_I$ = normal, Öl in Wasser ("normal, oil-in-water");

- $H_{II}$ = invertiert, Wasser in Öl ("inverted, water-in-oil") ;

- R = rhomboedrisch ("rhombohedral");

- Q = kubisch ("cubic");

- C = dreidimensional, kristallin ("three-dimensional, crystalline");

- $L_c$ = kristallin lamellar.

Die Phasen können beispielsweise mittels Röntgenbeugung, Freeze-Fracture Electron Microscopy oder $^{31}$P-NMR identifiziert werden.

Wenn das Fluid in einer derartigen geordneten Phase, insbesondere in einer eindimensional lamellaren Phase, vorliegt, kann die Bildung der Vesikel im Schritt b) unterstützt werden.

Die Komponenten des Fluides können vor Schritt a) mit an sich bekannten Verfahren vermischt werden.

Zur Charakterisierung der Strömung des Fluids kann der Geschwindigkeitsgradiententensor

$$L = \begin{pmatrix} \dfrac{\partial v_1}{\partial x_1} & \dfrac{\partial v_1}{\partial x_2} & \dfrac{\partial v_1}{\partial x_3} \\ \dfrac{\partial v_2}{\partial x_1} & \dfrac{\partial v_2}{\partial x_2} & \dfrac{\partial v_2}{\partial x_3} \\ \dfrac{\partial v_3}{\partial x_1} & \dfrac{\partial v_3}{\partial x_2} & \dfrac{\partial v_3}{\partial x_3} \end{pmatrix}$$

verwendet werden, wobei

$$\frac{\partial v_i}{\partial x_j}$$

die partielle räumliche Ableitung der Geschwindigkeitskomponente $v_i$ der Strömung in Richtung der Koordinatenachse $x_j$ bezeichnet. Dehnströmungen hängen generell mit der Beschleunigung

$$\frac{d\vec{v}}{dt}$$

eines Fluidelementes zusammen. Diese Beschleunigung ergibt sich bekanntermassen aus der Beziehung

$$\frac{d\vec{v}}{dt} = \frac{\partial \vec{v}}{\partial t} + \left(\vec{v}\vec{\nabla}\right)\vec{v} = \frac{\partial \vec{v}}{\partial t} + L\vec{v} \quad .$$

Im Falle einer stationären Strömung mit

$$\frac{\partial \vec{v}}{\partial t}$$

=0 ist die Beschleunigung also

$$\left(\vec{v}\vec{\nabla}\right)\vec{v} \ .$$

Auf der Grundlage dieser Erkenntnis wird der dimensionslose Parameter

$$K := \frac{|L\vec{v}|^2}{|\vec{v}|^2 \, \mathrm{sp}(LL^{\mathrm{T}})} = \frac{\left|\left(\vec{v}\vec{\nabla}\right)\vec{v}\right|^2}{|\vec{v}|^2 \, \mathrm{sp}(LL^{\mathrm{T}})} = \frac{\sum_j \left(\sum_i v_i \frac{\partial v_j}{\partial x_i}\right)^2}{\left(\sum_i v_i^2\right)\left(\sum_{i,j}\left(\frac{\partial v_j}{\partial x_i}\right)^2\right)}$$

definiert, wobei **sp(_LL_<sup>T</sup>)** die Spur des Matrixproduktes **_LL_<sup>T</sup>** von **_L_** mit dem zu **_L_** adjungierten Tensor **_L_<sup>T</sup>** ist. Mittels der Ausdrücke $|\vec{v}|^2$ und **sp(_LL_<sup>T</sup>)** im Nenner wird der Parameter **_K_** zu einer dimensionslosen Grösse normiert. Im Falle einer stationären, reinen Scherströmung, bei der die Fluidelemente nicht beschleunigt werden, ist der Parameter **_K_** gleich null. Enthält die Strömung Dehnströmungsanteile, so ist _K_ grösser als null. Aus diesem Grunde kann der Parameter **_K_** als Mass für den Anteil der Dehnströmung an der gesamten Strömung angesehen werden. Die obige Definition wird im Rahmen der Erfindung ebenfalls im Falle instationärer Strömungen verwendet - auch wenn dort

$$\left(\vec{v}\vec{\nabla}\right)\vec{v}$$

nicht gleich der Beschleunigung ist.

Zur Berechnung des Parameters **_K_** wird zunächst das Geschwindigkeitsvektorfeld $\bar{v}$ bestimmt. Dies kann durch näherungsweise numerische Lösung der Navier-Stokes-Gleichungen erfolgen, beispielsweise mit der Methode der Finiten Elemente, dem Finite-Volumen-Verfahren oder dem Finite-Differenzen-Verfahren. Zur Definition der Erfindung soll die Lösung mit dem Finite-Volumen-Verfahren erfolgen. Die Geometrie des Strömungsraumes, insbesondere der Begrenzungsflächen des Strömungsraumes, und die Geschwindigkeit des einströmenden Fluides dienen dabei als Randbedingen für die Lösung der Navier-Stokes-Gleichungen. Zur numerischen Berechnung mit dem Finite-Volumen-Verfahren steht eine Vielzahl an sich bekannter Software-Pakete zur Verfügung. Zur Berechnung im Rahmen der Erfindung soll jedoch die Software "CFX" der Firma ANSYS, Inc. verwendet werden. Der mit dem Finite-Volumen-Verfahren vertraute Fachmann ist in der Lage, die numerische Lösung mit dieser Software mit einer Genauigkeit von ± 10 % zu bestimmen.

Die räumlichen Ableitungen des Geschwindigkeitsvektorfeldes $\vec{v}$, die zur Bestimmung des Geschwindigkeitsgradententensor nötig sind, können ebenfalls mit an sich bekannten numerischen Methoden bestimmt werden.

Bevorzugt beträgt der Parameter **_K_** zumindest in dem Teilbereich des Strömungsraumes mindestens 0,3, bevorzugt mindestens 0,5, weiter bevorzugt mindestens 0,8, besonders bevorzugt mindestens 0,9.

Der symmetrische Anteil des Geschwindigkeitsgradententensors _L_,

$$D = \frac{1}{2}\left(L + L^{T}\right) = \begin{pmatrix} \dfrac{\partial v_1}{\partial x_1} & \dfrac{1}{2}\left(\dfrac{\partial v_1}{\partial x_2} + \dfrac{\partial v_2}{\partial x_1}\right) & \dfrac{1}{2}\left(\dfrac{\partial v_1}{\partial x_3} + \dfrac{\partial v_3}{\partial x_1}\right) \\[3mm] \dfrac{1}{2}\left(\dfrac{\partial v_1}{\partial x_2} + \dfrac{\partial v_2}{\partial x_1}\right) & \dfrac{\partial v_2}{\partial x_2} & \dfrac{1}{2}\left(\dfrac{\partial v_2}{\partial x_3} + \dfrac{\partial v_3}{\partial x_2}\right) \\[3mm] \dfrac{1}{2}\left(\dfrac{\partial v_1}{\partial x_3} + \dfrac{\partial v_3}{\partial x_1}\right) & \dfrac{1}{2}\left(\dfrac{\partial v_2}{\partial x_3} + \dfrac{\partial v_3}{\partial x_2}\right) & \dfrac{\partial v_3}{\partial x_3} \end{pmatrix} \ ,$$

wird als Verzerrungsgeschwindigkeitstensor bezeichnet. Als symmetrischer Tensor ist der Verzerrungsgeschwindigkeitstensor **D** mittels einer Hauptachsentransformation diagonalisierbar und hat im Hauptachsensystem drei Eigenwerte $\delta_1$, $\delta_2$ und $\delta_3$. Diese Eigenwerte charakterisieren den Grad der Deformation. Ein positiver Eigenwert stellt eine Dehnung in der zugehörigen Hauptachsenrichtung dar, während ein negativer Eigenwert eine Stauchung in der zugehörigen Hauptachsenrichtung darstellt.

Eine Dehnströmung wird als reine Dehnströmung bezeichnet, wenn jeweils einer der Eigenwerte des Verzerrungsgeschwindigkeitstensors positiv, negativ und null ist. Unter einer uniaxialen Dehnströmung wird eine Dehnströmung verstanden, bei der ein Eigenwert des Verzerrungsgeschwindigkeitstensors positiv ist und die beiden anderen negativ. Bei einer biaxialen Dehnströmung sind zwei Eigenwerte des Verzerrungsgeschwindigkeitstensors positiv und der dritte ist negativ. Eine biaxiale Dehnströmung führt zu einer erhöhten Vesikelbildung. Hingegen sind reine Dehnströmungen und uniaxiale Dehnströmungen technisch einfacher realisierbar. Das Fluid kann gemäss dem erfindungsgemässen Verfahren beispielsweise einer reinen Dehnströmung, einer uniaxialen Dehnströmung oder einer biaxialen Dehnströmung ausgesetzt werden.

Zur Charakterisierung des Dehnströmungsanteiles der Strömung ist weiterhin der dimensionslose Parameter

$$\tau^* = \int \dot{\gamma}_D \, dt$$

mit dem Integranden

$$\dot{\gamma}_D = \sqrt{2 \, \mathrm{sp}\left(D^2\right)}$$

geeignet, wobei $\mathrm{sp}(D^2)$ die Spur der Matrix $D^2 = DD$ bezeichnet. Die Integration wird über den Zeitraum ausgeführt, in dem sich ein Fluidteilchen innerhalb des Strömungsraums befindet. In vielen Ausführungsbeispielen hängt der Parameter $\tau^*$ von der betrachteten Stromlinie ab. Eine Stromlinie ist dabei eine imaginäre Linie, deren Tangente an jedem Punkt parallel zum Geschwindigkeitsvektorfeld ist.

In vorteilhaften Ausführungsformen liegt der Parameter $\tau^*$ für zumindest einen Teil der Stromlinien im Bereich von 1 bis 50, bevorzugt von 5 bis 40. Bevorzugt strömen mindestens 25 %, weiter bevorzugt mindestens 50 %, besonders bevorzugt mindestens 75 % der Durchflussmenge des Fluides entlang von Stromlinien, für die der Parameter $\tau^*$ in dem genannten Wertebereich liegt.

Für die Bildung von Vesikeln mittels eines Dehnströmungsanteils ist ein wesentlich kleinerer Wert des Para-meters $\tau^*$ ausreichend, als dies für den analogen Parameter einer Scherströmung der Fall ist. Mit anderen Worten genügt also bei einer Dehnströmung eine kürzere dimensionslose Zeit, um die Bildung von Vesikeln zu bewirken, als dies bei einer Scherströmung gleicher Grösse der Fall wäre. Die dimensionslose Zeit kann in einigen Fällen um einen Faktor zwischen 5 und 10 verringert werden. Folglich reichen für die Herstellung von Vesikeln mittels Dehnströmungen kleinere Volumina aus als für die Herstellung mittels Scherströmungen.

Die Erzeugung einer Strömung mit einem Dehnströmungsanteil in einem Fluid an sich ist dem Fachmann bekannt. Bevorzugt ist der Strömungsraum von mindestens einer Begrenzungsfläche begrenzt. Das Strömungsprofil, insbesondere der Dehnströmungsanteil und der optionale Scherströmungsanteil, kann durch die Geometrie des Strömungsraumes und insbesondere durch die Geometrie der mindestens einen Begrenzungsfläche festgelegt sein. Alternativ oder zusätzlich kann das Strömungsprofil auch durch die Bewegung mindestens einer Begrenzungsfläche festgelegt sein - beispielsweise durch die Oberflächen rotierender Walzen, wie unten im Detail beschrieben. Ebenfalls alternativ oder zusätzlich kann das Strömungsprofil durch eine Pumpe festgelegt sein, welche das Fluid in den Strömungsraum hinein oder innerhalb des Strömungsraumes befördert. Zudem kann das Strömungsprofil durch ein Erwärmen der Begrenzungsfläche beeinflusst werden. Durch eine Erwärmung kann nämlich die Viskosität des Fluides im Bereich der Begrenzungsflächen erniedrigt werden, wodurch geringere Reibungskräfte zwischen dem Fluid und den Begrenzungsflächen auftreten. Der Scherströmungsanteil kann hierdurch reduziert werden, was zu einer gleichmässigeren Grössenverteilung der erzeugten Vesikel führt.

In einigen Ausführungsformen wird die Begrenzungsfläche während der Durchführung des Verfahrens nicht bewegt. Es kann somit eine Vorrichtung mit unbeweglichen Begrenzungsflächen verwendet werden, wodurch der Verschleiss der Vorrichtung herabgesetzt wird. In bevorzugten Ausführungsformen weist der Strömungsraum einen Querschnitt auf, der in einer Strömungsrichtung des Fluides veränderlich ist. Bevorzugt nimmt der Querschnitt in der Strömungsrichtung ab, so dass eine Verengung im Strömungsraum entsteht. Hierdurch kann eine uniaxiale Dehnströmung erzeugt werden. Beispielsweise kann der Strömungsraum als Düse ausgebildet sein. Alternativ ist es auch möglich, dass der Querschnitt in der Strömungsrichtung zunimmt, so dass eine Aufweitung im Strömungsraum vorliegt. Hierdurch kann eine biaxiale Dehnströmung erzeugt werden. Es ist auch denkbar, dass der Querschnitt in einem oder mehreren Teilen des Strömungsraumes abnimmt und in einem oder mehreren anderen Teilen des Strömungsraumes zunimmt. Der Querschnitt kann durch die mindestens eine Begrenzungsfläche definiert sein. Hierdurch kann ohne bewegliche Begrenzungsflächen ein grosser Dehnströmungsanteil erzeugt werden.

In einigen Ausführungsformen können die Begrenzungsflächen teilweise durch die Oberflächen von Schikanen gebildet sein, welche innerhalb des Strömungsraumes angeordnet sind. Derartige Schikanen können zu einer Querschnittsverengung führen, welche ihrerseits Dehnströmungsanteile im Fluid erzeugen und damit die Bildung von Vesikeln bewirken. Die Schikanen können als Schüttgut ausgebildet sein, welches im Strömungsraum angeordnet ist. Bei dem Schüttgut kann es sich beispielsweise um ein Granulat handeln. Wenn das Fluid durch die Zwischenräume zwischen den einzelnen Partikeln des Schüttguts strömt, erfährt es Dehnströmungsanteile, welche die Erzeugung von Vesikeln bewirken.

Das Granulat kann beispielsweise kugelförmig sein. Andere Formen sind jedoch ebenfalls denkbar und liegen im Rahmen der Erfindung. Das Granulat kann Partikel mit Grössen, insbesondere mit Kugeldurchmessern, im Bereich von 0,1 mm bis 3 mm, bevorzugt von 0,3 mm bis 1 mm aufweisen. Das Granulat kann beispielsweise aus Metall, Kunststoff oder Glas bestehen.

Der Strömungsraum kann von einem Strömungsrohr begrenzt sein, in welchem das Granulat angeordnet ist. Das Strömungsrohr kann beispielsweise vertikal ausgerichtet und im Wesentlichen zylindrisch sein.

Optional kann die Strömung des Fluides instationär sein. Hierunter wird verstanden, dass die zeitliche partielle Ableitung

$$\frac{\partial \bar{v}}{\partial t}$$

an einem festen Raumpunkt innerhalb des Strömungsraumes ungleich null ist; die Berechnung erfolgt also in der Eulerschen Betrachtungsweise. Hierdurch werden Trägheitskräfte wirksam, welche die Bildung von Vesikeln beeinflussen und insbesondere erleichtern können.

In einigen Ausführungsformen wird das Fluid mittels Ultraschalls in mechanische Schwingungen versetzt und/oder mit mechanischen Wellen beaufschlagt. Der Ultraschall kann mit einem an sich bekannten Ultraschall-Resonator erzeugt werden.

Das Verfahren kann in einer an sich bekannten Vorrichtung durchgeführt werden. Beispielsweise kann die Vorrichtung eine Düse enthalten, welche den Strömungsraum bildet. Alternativ kann es sich bei der Vorrichtung um eine Spaltmühle handeln, wie sie beispielsweise in WO 98/00228 beschrieben ist.

Die Vorrichtung kann, wie in WO 98/00228 offenbart, einen Einlass für das Fluid, mindestens einen Strömungsraum und mindestens einen Auslass für das Fluid aufweisen. Das aus dem Auslass ausströmende Fluid enthält die durch die Einwirkung der Dehnströmung erzeugten Vesikel. Weiterhin kann die Vorrichtung eine Pumpe enthalten, mittels welcher das Fluid gefördert und die Strömung erzeugt wird.

In einer möglichen Ausführungsform kann die Vorrichtung zwei gegeneinander gerichtete Grundkörper enthalten, zwischen denen der Strömungsraum gebildet ist. Die Teile der Oberflächen der Grundkörper, die dem jeweils anderen Grundkörper zugewandt sind, bilden in diesem Falle die Begrenzungsflächen des Strömungsraumes. Die Begrenzungsflächen der beiden Grundkörper können jeweils einen mittleren Bereich und einen äusseren Bereich enthalten. In den mittleren Bereichen können die Begrenzungsflächen einen kleineren Abstand voneinander haben als in den äusseren Bereichen. Wenn ein Fluid im Strömungsraum vom äusseren Bereich in Richtung des inneren Bereiches strömt, d. h. in radialer Richtung von aussen nach innen, so definieren die Begrenzungsflächen in der Strömungsrichtung einen abnehmenden Querschnitt. Die Grundkörper können zu diesem Zweck beispielsweise konvex sein, so dass die Begrenzungsflächen insbesondere konusförmig oder kugelschalenförmig sein können.

Die im Fluid enthaltene amphiphile Substanz kann je nach den Erfordernissen gewählt werden. Derartige Substanzen sind dem Fachmann an sich bekannt. Die amphiphile Substanz kann beispielsweise ausgewählt sein aus der Gruppe, die besteht aus Tensiden, Emulgatoren, insbesondere Lecithinen wie etwa Soja-Lecithin, Lipiden, insbesondere Phospholipiden, und Block-Copolymeren. Spezielle Ausführungsformen dieser Substanzen, insbesondere spezielle Block-Copolymere, sowie weitere mögliche amphiphile Substanzen sind in der oben genannten europäischen Patentanmeldung EP 10159108.9 offenbart; auf diese amphiphilen Substanzen wird hiermit Bezug genommen.

Das Fluid kann ein Lösungsmittel enthalten, wie beispielsweise Wasser. Weiterhin kann das Fluid mindestens eine Pufferlösung und/oder mindestens ein Tensid und/oder mindestens ein Salz und/oder mindestens eine Säure und/oder mindestens eine Lauge umfassen. Hierdurch kann die Stabilität der Vesikel eingestellt werden und insbesondere der Zeitraum definiert werden, innerhalb dessen ein im Vesikel enthaltener Wirkstoff freigesetzt wird. Das Fluid kann kompressibel oder inkompressibel sein.

Weiterhin bevorzugt umfasst das Fluid mindestens einen insbesondere zu verkapselnden Wirkstoff. Dieser kann beispielsweise im Fluid suspendiert oder gelöst sein. Unter einem Wirkstoff wird hier und im Folgenden eine Substanz verstanden, die in geringer Dosis in einem Organismus eine spezifische Wirkung hervorruft. Der Wirkstoff kann ein niedermolekularer Stoff sein, beispielsweise ein Arzneistoff, oder auch ein hochmolekularer Stoff, beispielsweise ein Protein. Beim erfindungsgemäßen Verfahren kann somit die gesonderte Verkapselung oder Umhüllung des mindestens einen Wirkstoffs entfallen. Hierdurch können aufwendige Techniken, wie z. B. "remote loading" über einen pH-Gradienten, Hochdruckhomogenisierung oder "ethanol-injection method" (Injektion einer Ethanol-Lipid-Lösung in die Wirkstofflösung) vermieden werden.

Ein weiterer Aspekt der Erfindung betrifft eine Vorrichtung zur Herstellung von Vesikeln und/oder zum Dispergieren, Desagglomerieren, Mischen und/oder Emulgieren eines Fluides. Die Vorrichtung kann beispielsweise zur Durchführung des erfindungsgemässen Verfahrens geeignet sein. Die Vorrichtung umfasst mindestens ein Paar von drehbaren Walzen mit jeweils einer im Wesentlichen zylindermantelförmigen Walzfläche. Zwischen den Walzen ist ein Strömungsraum für ein Fluid gebildet. Erfindungsgemäss weist mindestens eine der Walzen einen Hohlraum auf und weist die Walzfläche dieser Walze mindestens eine Einströmöffnung auf, durch welche ein Fluid vom Strömungsraum in den Hohlraum strömen kann. Bevorzugt umfasst die Walzfläche mindestens eine Membran, welche die Einströmöffnungen enthält.

Durch eine gegenläufige Rotation der Walzen kann ein Fluid, beispielsweise ein wie oben beschriebenes Fluid mit mindestens einer amphiphilen Substanz, in den Zwischenraum zwischen den Walzen strömen, der somit einen Strömungsraum für das Fluid bildet. Diese Strömung kann durch die eigene Schwerkraft des Fluides unterstützt werden. Aufgrund der Rotation der Walzen entsteht im Zwischenraum zwischen den Walzen ein Dehnströmungsanteil in der Strömung, der die Bildung von Vesikeln aus dem Fluid bewirkt. Das die Vesikel enthaltende Fluid kann dann durch die Einströmöffnung(en) in den Hohlraum der Walze strömen. Je einheitlicher die Grössenverteilung der Einströmöffnungen ist, desto einheitlicher ist auch die Grössenverteilung der erzeugten Vesikel.

Die Walzen können mit derartigen Geschwindigkeiten rotieren, dass sich die Oberflächen der Walzen mit der gleichen Geschwindigkeit bewegen. Im Falle zweier Walzen mit gleichen Radien müssen dazu die Drehgeschwindigkeiten beider Walzen übereinstimmen. Es ist jedoch auch denkbar und liegt im Rahmen der Erfindung, dass die Walzen mit derartigen Geschwindigkeiten rotieren, dass sich die Oberflächen der Walzen mit verschiedenen Geschwindigkeiten und daher mit einer von null verschiedenen zueinander bewegen. Durch eine von null verschiedene Relativgeschwindigkeit der Oberflächen können zusätzlich Scherströmanteile im Fluid erzeugt werden. Beispielsweise kann die Geschwindigkeit der Oberfläche der einen Walze um bis zu 5 % grösser oder kleiner sein als die Geschwindigkeit der Oberfläche der anderen Walze.

Die Walzfläche kann etwa ein poröses Metall, ein poröses Sintermaterial und/oder einen porösen Polymerfilm umfassen. Die Walzfläche kann auch mehrere Schichten umfassen. Beispielsweise kann sie einen porösen Sinterkörper enthalten, welcher mit einem Polymerfilm umwickelt oder überzogen ist. Die Einströmöffnungen können beispielsweise durch Sintern, Ätzen, Laserbohren, Bestrahlen, z. B. mit Röntgenstrahlung, oder durch Partikelbeschuss, z. B. mit Elektronen, in die Walzfläche eingebracht werden. Sie können Durchmesser im Bereich von $0{,}1\ \mu m$ bis $20\ \mu m$, bevorzugt von $0{,}2\ \mu m$ bis $1\ \mu m$, besonders bevorzugt von $0{,}4\ \mu m$ bis $0{,}6\ \mu m$ haben. Die Dicke der Walzfläche definiert die Länge der Einströmöffnungen und kann das 1-fache bis 100-fache des Durchmessers des Einströmöffnungen betragen.

Bevorzugt weist die Walze insbesondere im Bereich einer Stirnseite mindestens eine Ausströmöffnung auf, durch welche das Fluid aus dem Hohlraum ausströmen kann. Hierdurch kann das Vesikel enthaltende Fluid leicht entnommen und beispielsweise weiterverarbeitet oder zur Lagerung abgefüllt werden.

Die Erfindung wird nachfolgend anhand von mehreren Ausführungsbeispielen und Zeichnungen näher erläutert. Es zeigen

Figur 1:            eine Prinzipskizze einer ersten Ausführungsform einer Vorrichtung zur Durchführung des Verfahrens;

Figur 2:            eine Prinzipskizze einer zweiten Ausführungsform einer Vorrichtung zur Durchführung des Verfahrens;

Figuren 3a und 3b:      eine Prinzipskizze einer dritten Ausführungsform einer Vorrichtung zur Durchführung des Verfahrens;

Figur 4:            eine Prinzipskizze einer vierten Ausführungsform einer Vorrichtung zur Durchführung des Verfahrens;

Figur 5:            eine Prinzipskizze einer fünften Ausführungsform einer Vorrichtung zur Durchführung des Verfahrens.

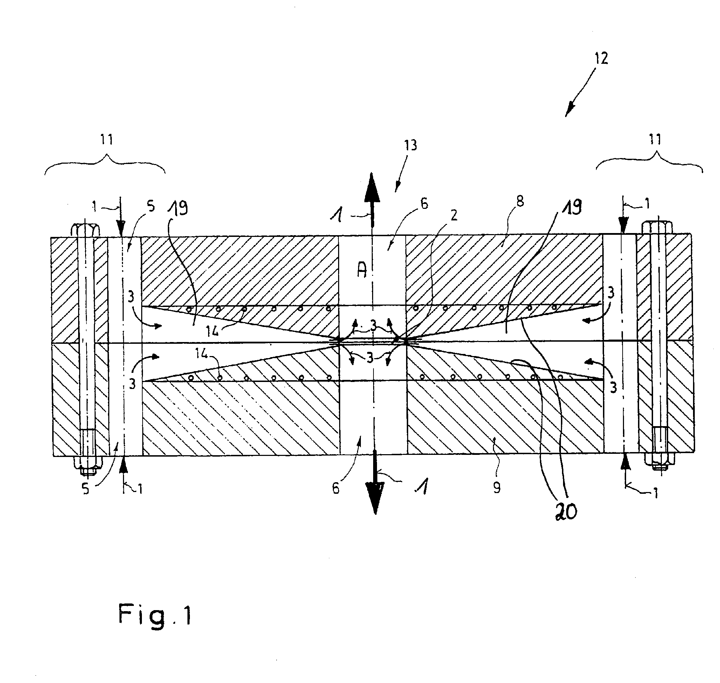

Die in Fig. 1 nicht massstabsgetreu dargestellte Vorrichtung 12 weist zwei gegeneinander gerichtete Grundkörper 8 und 9 auf, die jeweils ein- oder mehrstückig ausgebildet sein können. Jeder der beiden Grundkörper 8 und 9 weist eine konusförmige Oberfläche 20 auf, deren Symmetrieachse mit A bezeichnet ist und die einen halben Öffnungswinkel von etwa 81˚ aufweist. Diese Oberflächen 20 bilden Begrenzungsflächen für einen Strömungsraum 19, der zwischen den Grundkörpern 8, 9 gebildet ist. Die Grundkörper 8,9 haben jeweils einen Durchmesser von 10 cm.

In einem Aussenbereich 11 der Grundkörper 8 und 9 befinden sich Bohrungen 5 für den Einlass eines Fluides 1. Die Bohrungen 5 sind gleichmässig um die Symmetrieachse A herum angeordnet; Figur 1 zeigt eine Schnittansicht durch zwei dieser Bohrungen 5. Alternativ kann aber auch nur eine einzige verbundene, ringförmige Bohrung 5 vorhanden sein, welche die Symmetrieachse A umschliesst. Mögliche Zusammensetzungen des Fluides 1 sind unten beschrieben. Im Zentrum 13 der Vorrichtung 12 befinden sich Bohrungen 6 für den Auslass des Vesikel enthaltenden Fluides 1.

Die Geometrie der Begrenzungsflächen 20 legt die Strömung des Fluides 1 fest. Es bildet sich eine ortsabhängige Strömungsgeschwindigkeit 3. Die Begrenzungsflächen 20 sind so ausgebildet, dass der Querschnitt des Strömungsraumes 19 in der Strömungsrichtung 3, also in radialer Richtung nach innen, abnimmt. Hierdurch wird eine Verengung 2 bebildet, aus der die Bohrungen 6 für den Auslass führen. Die Verengung 2 hat eine Breite von 0,3 mm; die Bohrungen 6 haben Durchmesser von 5 mm. Durch diese Querschnittsverengung 2 entsteht ein Dehnströmungsanteil in der Strömung, welcher die Bildung von Vesikeln aus dem Fluid bewirkt.

Bei Verwendung eines Fluides mit der unten beschriebenen Zusammensetzung und einer Temperatur von 20 ˚C liegt der Parameter $\tau^*$ liegt im Bereich von 20 bis 40. Diese Grösse ist praktisch unabhängig von der Durchflussmenge des Fluides 1. Wie oben bereits erläutert wurde, ist dieser Parameter von der betrachteten Stromlinie abhängig.

Vorzugsweise können die Grundkörper 8 und/oder 9 mit Heiz-und/oder Kühlelementen 14 versehen sein. Heizelemente 14 können die Temperatur des Fluides 1 an den Begrenzungsflächen 20 erhöhen und hierdurch seine Viskosität erniedrigen. Infolgedessen treten geringere Reibungskräfte zwischen dem Fluid 1 und den Begrenzungsflächen 20 auf. Der Scherströmungsanteil wird hierdurch weiter reduziert, was wiederum zu einer gleichmässigeren Grössenverteilung der erzeugten Vesikel führt. Im weiteren Aussenbereich sind Befestigungsmittel 15 zum Zusammenhalten der Grundkörper 8 und 9 vorgesehen.

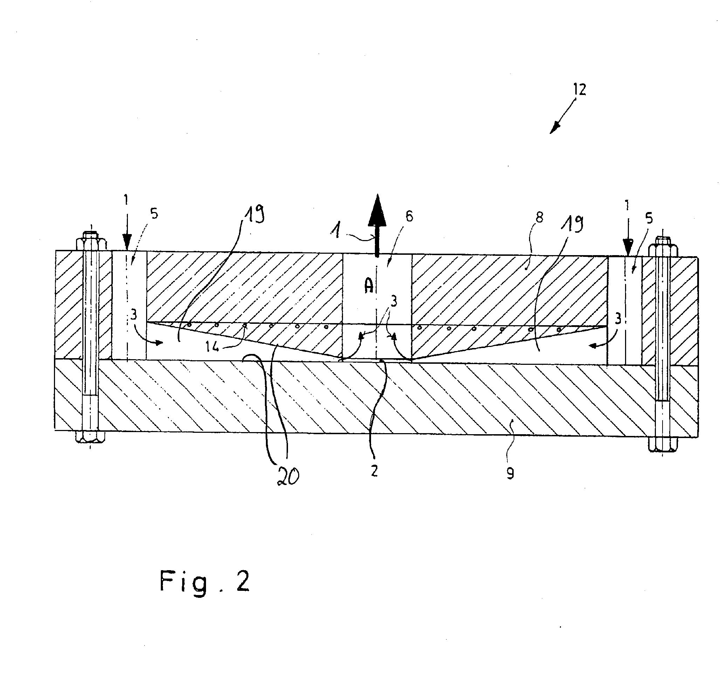

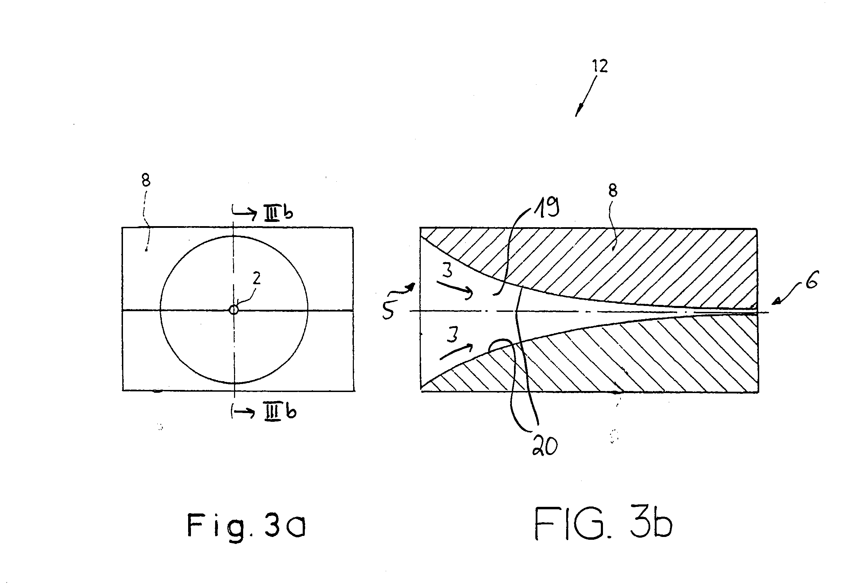

Die Figuren 2 und 3a/3b zeigen weitere Ausführungsformen der Vorrichtung 12. In allen Figuren werden dieselben Bezugszeichen verwendet.

Bei der zweiten Ausführungsform gemäss Figur 2 ist im Gegensatz zur ersten Ausführungsform gemäss Figur 1 der Grundkörper 9 als ebene Platte ausgebildet, deren Oberfläche 20 eine ebene Begrenzungsfläche des Strömungsraumes 19 bildet. Wie bei der ersten Ausführungsform nimmt der Querschnitt des Strömungsraumes 19 in der Strömungsrichtung 3, also in radialer Richtung nach innen, ab. Durch diese Querschnittsverengung entsteht ein Dehnströmungsanteil in der Strömung, welcher die Bildung von Vesikeln aus dem Fluid bewirkt.

Die dritte Ausführungsform gemäss Figuren 3a und 3b enthält einen einstückig ausgebildeten Grundkörper 8 in Form einer Düse, die in Figur 3a in einer Draufsicht und in Figur 3b in einer Schnittansicht dargestellt ist. Die innere Oberfläche 20 des Grundkörpers 8 bildet eine Begrenzungsfläche, die einen Strömungsraum 19 mit einem sich in Strömungsrichtung 3 verengenden Querschnitt begrenzt. Durch diese Querschnittsverengung entsteht ein Dehnströmungsanteil, welcher die Bildung von Vesikeln aus dem Fluid bewirkt. Die Vorrichtung gemäss Figuren 3a und 3b ist aufgrund ihrer Bauweise wesentlich einfacher herstellbar als die in den Figuren 1 und 2 gezeigten.

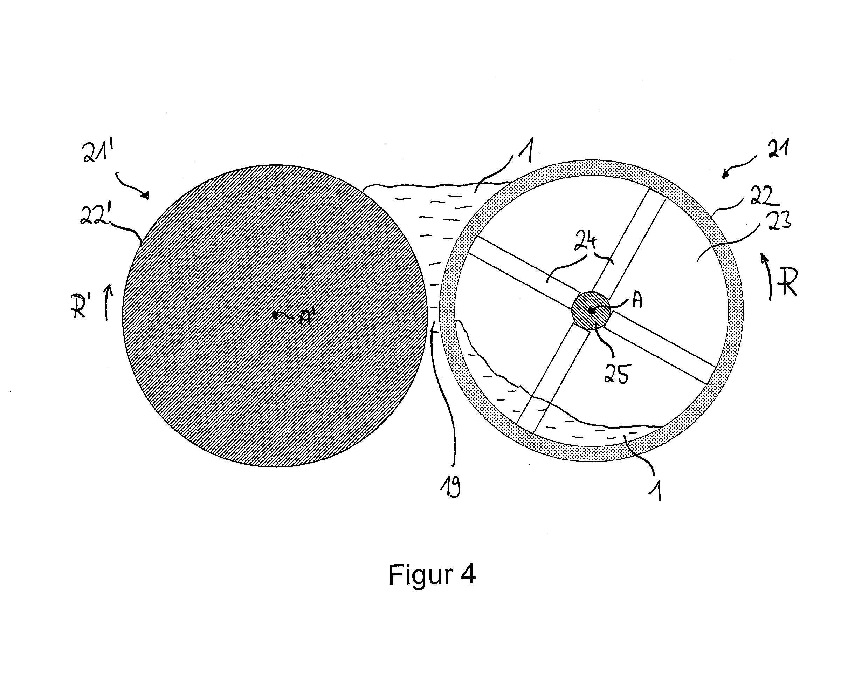

Figur 4 zeigt eine Schnittansicht einer vierten Ausführungsform der Vorrichtung. Die Vorrichtung enthält ein Paar von Walzen 21, 21' mit jeweils einer zylindermantelförmigen Walzfläche 22, 22' mit einem Radius von 30 cm. Die Walzen 21, 21' können in zueinander entgegengesetzten Drehrichtungen R, R' um jeweilige zueinander parallele und horizontal ausgerichtete Drehachsen A, A' gedreht werden. Die Walzen 21, 21' mit der gleichen Drehgeschwindigkeit rotieren, so dass die Walzflächen 22, 22' keine Relativgeschwindigkeit aufweisen. Alternativ können die Walzen auch mit verschiedenen Drehgeschwindigkeiten rotieren, so zwischen beiden Walzflächen 22, 22' eine von null verschiedene Relativgeschwindigkeit auftritt. Die rechts dargestellte Walze 21 weist einen Hohlraum 23 auf, welcher von der Walzfläche 22 in radialer Richtung umschlossen ist. Die Walzfläche 22 ist über vier Speichen 24 mit einer zentralen Welle 25 verbunden.

Die Walzfläche 22 weist mehrere in der Abbildung nicht erkennbare Einströmöffnungen auf. Die Walzfläche 22 besteht aus einem Sintermetall und hat eine Dicke von 2 cm. Optional kann das Sintermetall mit einem porösem Polymerfilm umwickelt sein. Die Einströmöffnungen können beispielsweise durch Bohren in die Walzfläche 22 eingebracht werden und haben einen Durchmesser von 200 μm.

Zwischen den beiden Walzen 21, 21' befindet sich ein Zwischenraum 19. In diesen Zwischenraum 19 wird von oben ein Fluid 1 aus einer nicht dargestellten Zuführvorrichtung bereitgestellt. Das Fluid 1 enthält eine amphiphile Substanz und kann die unten angegebene Zusammensetzung haben. Aufgrund der Rotation der Walzen 21, 21' und aufgrund der Schwerkraft des Fluides 1 dringt dieses in den Zwischenraum 19 ein, welcher daher einen Strömungsraum für das Fluid 1 bildet. Die durch die Drehung der Walzen 21, 21' und die Schwerkraft erzeugten Dehnströmungsanteile bewirken die Bildung von Vesikeln aus dem Fluid 1.

Ein Teil des die Vesikel enthaltenden Fluides 1 strömt aufgrund des zwischen den Walzflächen 22, 22' bestehenden Druckes durch die Einströmöffnungen der Walzfläche 22 in den Hohlraum 23. Der Abstand zwischen den Walzen 21, 21' ist so eingestellt, dass der wesentliche Anteil des nicht in den Hohlraum 23 eintretenden Fluides 1 an den Walzflächen 22, 22' kleben bleibt und nach einer Umdrehung erneut in den Zwischenraum 19 zwischen den Walzen 21, 21' geführt wird.

Der in den Hohlraum 23 gepresste Anteil des Fluides 1 läuft aufgrund seiner Oberflächenspannung an der Unterseite der Walzfläche 22 nicht wieder aus dem Hohlraum 23 hinaus. Stattdessen kann das Fluid 1 durch eine hier nicht dargestellte Austrittsöffnung aus dem Hohlraum 23 ausströmen, die im Bereich einer Stirnseite der Walze 21 angeordnet ist.

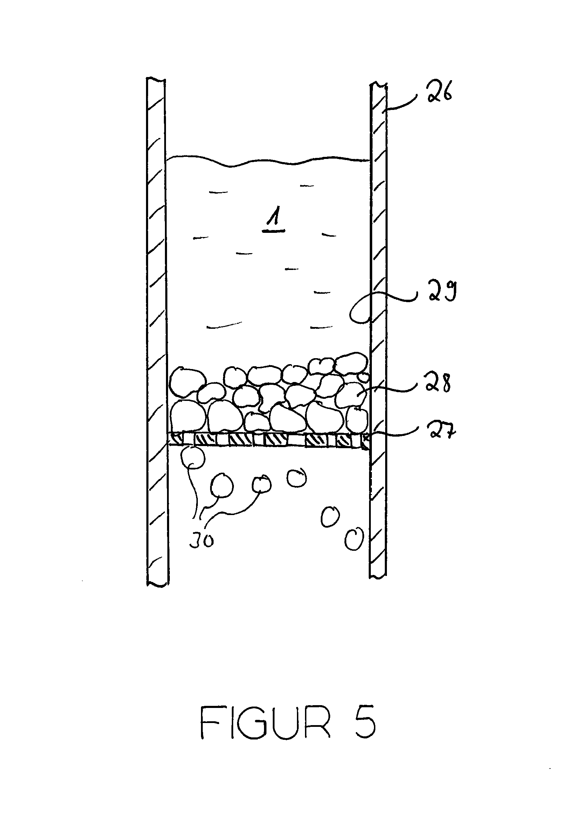

Schliesslich zeigt Figur 5 eine fünfte Ausführungsform einer Vorrichtung zur Durchführung des Verfahrens. Der Strömungsraum 19 wird von einem zylindrischen und vertikal ausgerichteten Strömungsrohr 26 begrenzt. Im Strömungsrohr 26 ist ein Gitter 27 angeordnet, auf dem ein Granulat 28 ruht. Das Granulat 28 kann beispielsweise aus Metall bestehen und von Partikelgrössen von 0,3 mm bis 1 mm aufweisen. Sowohl die Innenwand 29 des Strömungsrohres 26 als auch die Oberflächen des Granulates 28 bilden Begrenzungsflächen des Strömungsraumes 19. Ein Fluid 1 wird von oben in das Strömungsrohr 19 eingeleitet und strömt aufgrund seiner Schwerkraft durch die Zwischenräume zwischen den einzelnen Partikeln des Granulates 28, wobei es Dehnströmungsanteile erfährt, welche die Erzeugung von Vesikeln bewirken. Unterhalb des Gitters 27 bilden sich Tropfen 30 des Fluides 1, welche die Vesikel enthalten.

Das in den obigen Ausführungsbeispielen verwendete Fluid 1 kann beispielsweise 30 bis 40 Gew.-% Lecithin, wie etwa Soja-Lecithin, und Wasser enthalten. Optional kann das Fluid auch Wirkstoffe enthalten, wie beispielsweise Vitamin C. Die Komponenten können durch an sich bekannte Verfahren miteinander vermischt werden.

**Patentansprüche**

1.   Verfahren zur Herstellung von Vesikeln, umfassend die folgenden Schritte:

   a) Bereitstellen eines Fluides (1), welches zumindest eine amphiphile Substanz enthält;
   b) Erzeugung einer Strömung des Fluides (1) in mindestens einem Strömungsraum (19), wobei die Strömung wenigstens in einem Teilbereich des Strömungsraumes (19) zumindest einen Dehnströmungsanteil enthält, der die Bildung von Vesikeln aus dem Fluid (1) bewirkt.

2.   Verfahren gemäss Anspruch 1,
   **dadurch gekennzeichnet, dass**
   das Volumen des Teilbereiches des Strömungsraumes (19) mindestens 25 %, bevorzugt in mindestens 50 %, besonders bevorzugt in mindestens 75 % des Volumens des gesamten Strömungsraumes (19) beträgt.

3.   Verfahren gemäss einem der vorangehenden Ansprüche,
   **dadurch gekennzeichnet, dass**
   das in Schritt a) bereitgestellte Fluid (1) in einer geordneten Phase vorliegt.

4.   Verfahren gemäss einem der vorangehenden Ansprüche,

**dadurch gekennzeichnet, dass**
der Parameter

$$K := \frac{\left|L\vec{v}\right|^2}{\left|\vec{v}\right|^2 \mathrm{sp}\!\left(LL^{\mathrm{T}}\right)} = \frac{\left|\left(\vec{v}\vec{\nabla}\right)\vec{v}\right|^2}{\left|\vec{v}\right|^2 \mathrm{sp}\!\left(LL^{\mathrm{T}}\right)} = \frac{\sum_j \left(\sum_i v_i \dfrac{\partial v_j}{\partial x_i}\right)^2}{\left(\sum_i v_i^{\,2}\right)\!\left(\sum_{i,j}\left(\dfrac{\partial v_j}{\partial x_i}\right)^2\right)}$$

zumindest in dem Teilbereich des Strömungsraumes (19) mindestens 0,3, bevorzugt mindestens 0,5, weiter bevorzugt mindestens 0,8, besonders bevorzugt mindestens 0,9 beträgt.

5. Verfahren gemäss einem der vorangehenden Ansprüche,
   **dadurch gekennzeichnet, dass**
   der Parameter $\tau^* = \int \dot{\gamma}_{\mathrm{D}}\,dt$ für zumindest einen Teil der Stromlinien im Bereich von 1 bis 50, bevorzugt von 5 bis 40 liegt.

6. Verfahren gemäss Anspruch 5,
   **dadurch gekennzeichnet, dass**
   mindestens 25 %, weiter bevorzugt mindestens 50 %, besonders bevorzugt mindestens 75 % der Durchflussmenge des Fluides (1) entlang von Stromlinien strömt, für die der Parameter $\tau^*$ im Bereich von 1 bis 50, bevorzugt von 5 bis 40 liegt.

7. Verfahren gemäss einem der vorangehenden Ansprüche,
   **dadurch gekennzeichnet, dass**
   der Strömungsraum (19) von mindestens einer Begrenzungsfläche (20) begrenzt ist, die insbesondere während der Durchführung des Verfahrens nicht bewegt wird.

8. Verfahren gemäss einem der vorangehenden Ansprüche,
   **dadurch gekennzeichnet, dass**
   der Strömungsraum (19) einen Querschnitt aufweist, der in einer Strömungsrichtung (3) des Fluides (1) veränderlich ist, insbesondere abnimmt und/oder zunimmt.

9. Verfahren gemäss einem der vorangehenden Ansprüche,
   **dadurch gekennzeichnet, dass**
   die Strömung des Fluides instationär ist.

10. Verfahren gemäss einem der vorangehenden Ansprüche,
    **dadurch gekennzeichnet, dass**
    die amphiphile Substanz ausgewählt ist aus der Gruppe, die besteht aus Tensiden, Emulgatoren, insbesondere Lecithinen wie etwa Soja-Lecithin, Lipiden, insbesondere Phospholipiden, und Block-Copolymeren.

11. Verfahren gemäss einem der vorangehenden Ansprüche,
    **dadurch gekennzeichnet, dass**
    das Fluid einen insbesondere zu verkapselnden Wirkstoff enthält.

12. Vorrichtung zur Herstellung von Vesikeln, insbesondere in einem Verfahren gemäss einem der vorangehenden Ansprüche, und/oder zum Dispergieren, Desagglomerieren, Mischen und/oder Emulgieren eines Fluides, umfassend mindestens ein Paar von drehbaren Walzen (21,21') mit jeweils einer im Wesentlichen zylindermantelförmigen Walzfläche (22,22'), wobei zwischen den Walzen (21,21') ein Strömungsraum (19) für ein Fluid (1) gebildet ist,
    **dadurch gekennzeichnet, dass**
    mindestens eine der Walzen (21) einen Hohlraum (23) aufweist und die Walzfläche (22) dieser Walze (21) mindestens eine Einströmöffnung aufweist, durch welche ein Fluid (1) vom Strömungsraum (19) in den Hohlraum (23) strömen kann.

13. Vorrichtung gemäss Anspruch 12,
**dadurch gekennzeichnet, dass**
die Walze (21) insbesondere im Bereich einer Stirnseite mindestens eine Ausströmöffnung aufweist, durch welche das Fluid (1) aus dem Hohlraum (23) ausströmen kann.

14. Vorrichtung gemäss einem der Ansprüche 12 und 13,
**dadurch gekennzeichnet, dass**
die Walzfläche (22) ein poröses Metall, ein poröses Sintermaterial und/oder oder einen porösen Polymerfilm umfasst.

15. Vorrichtung gemäss einem der Ansprüche 12 bis 14,
**dadurch gekennzeichnet, dass**
die mindestens eine Einströmöffnung einen Durchmesser im Bereich von 0,1 μm bis 20 μm, bevorzugt von 0,2 μm bis 1 μm, besonders bevorzugt von 0,4 μm bis 0,6 μm hat.

Fig.1

EP 2 402 075 A1

Fig . 2

FIG. 3b

Fig. 3a

Figur 4

FIGUR 5

EP 2 402 075 A1

**Europäisches Patentamt**
**European Patent Office**
**Office européen des brevets**

# EUROPÄISCHER RECHERCHENBERICHT

Nummer der Anmeldung

EP 10 16 7570

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | EP 1 920 765 A1 (MEDIGENE AG [DE]; UNIV MUENCHEN L MAXIMILIANS [DE]) 14. Mai 2008 (2008-05-14) * Ansprüche 1, 6, 14, 23 * * Abbildung 2 * * Absätze [0032], [0033], [0042] * ----- | 1-11 | INV. B01J13/04 A61K9/50 |
| A | EP 0 649 867 A1 (UNILEVER PLC [GB]) 26. April 1995 (1995-04-26) * das ganze Dokument * ----- | 1-11 | |
| X | WO 2008/009623 A1 (NESTEC SA [CH]; WINDHAB ERICH JOSEF [CH]; MUELLER-FISCHER NADINA PATRI) 24. Januar 2008 (2008-01-24) * Anspruch 9 * * Seite 6, Zeile 8 - Zeile 15 * * Seite 6, Zeile 19 - Zeile 22 * * Seite 45, Zeile 13 - Zeile 29 * * Abbildung 12 * ----- | 12-15 | |
| X | DE 20 54 968 A1 (TITUS, HANS-JOACHIM) 10. Mai 1972 (1972-05-10) * Ansprüche 1-2 * * Abbildung 1 * ----- | 12-15 | RECHERCHIERTE SACHGEBIETE (IPC) B01J A61K |
| X | FR 2 652 762 A1 (LECOFFRE YVES [FR]) 12. April 1991 (1991-04-12) * Ansprüche 1,5 * * Abbildung 1 * ----- | 12-15 | |
| X | DATABASE WPI Week 198934 Thomson Scientific, London, GB; AN 1989-247485 XP002637417, & SU 1 442 258 A (ROST AGRIC ENG INST) 7. Dezember 1988 (1988-12-07) * Zusammenfassung * ----- -/-- | 12-15 | |

Der vorliegende Recherchenbericht wurde für alle Patentansprüche erstellt

| Recherchenort | Abschlußdatum der Recherche | Prüfer |
|---|---|---|
| Den Haag | 31. Mai 2011 | Tarallo, Anthony |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

17

Europäisches
Patentamt

European
Patent Office

Office européen
des brevets

# EUROPÄISCHER RECHERCHENBERICHT

**Nummer der Anmeldung**

EP 10 16 7570

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | DATABASE WPI<br>Week 197913<br>Thomson Scientific, London, GB;<br>AN 1979-25485B<br>XP002637418,<br>& SU 606 609 A (LENGD CHEM PHARM)<br>13. April 1978 (1978-04-13)<br>* Zusammenfassung *<br>----- | 12-15 | |
| X | DATABASE WPI<br>Week 199817<br>Thomson Scientific, London, GB;<br>AN 1998-192065<br>XP002637419,<br>& RU 2 088 870 C1 (CONSERVE & FRUIT DRYING IND INST) 27. August 1997 (1997-08-27)<br>* Zusammenfassung *<br>----- | 12-15 | |
| X | DATABASE WPI<br>Week 199406<br>Thomson Scientific, London, GB;<br>AN 1994-046415<br>XP002637420,<br>& RU 2 000 067 C (KVASENKOV O I)<br>7. September 1993 (1993-09-07)<br>* Zusammenfassung *<br>----- | 12-15 | RECHERCHIERTE SACHGEBIETE (IPC) |
| A | US 5 312 544 A (KINNEY CRAIG S [US])<br>17. Mai 1994 (1994-05-17)<br>* das ganze Dokument *<br>----- | 12-15 | |

-/--

Der vorliegende Recherchenbericht wurde für alle Patentansprüche erstellt

| Recherchenort | Abschlußdatum der Recherche | Prüfer |
|---|---|---|
| Den Haag | 31. Mai 2011 | Tarallo, Anthony |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus anderen Gründen angeführtes Dokument

 

& : Mitglied der gleichen Patentfamilie, übereinstimmendes
    Dokument

EPO FORM 1503 03.82 (P04C03)

| Europäisches Patentamt European Patent Office Office européen des brevets | **EUROPÄISCHER RECHERCHENBERICHT** | **Nummer der Anmeldung**<br>EP 10 16 7570 |
|---|---|---|

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | SCHADLER V ET AL: "Continuous membrane emulsification by using a membrane system with controlled pore distance", DESALINATION, ELSEVIER, AMSTERDAM, NL, Bd. 189, Nr. 1-3, 1. März 2006 (2006-03-01), Seiten 130-135, XP025161377, ISSN: 0011-9164, DOI: DOI:10.1016/J.DESAL.2005.06.020 [gefunden am 2006-03-01] * das ganze Dokument * ----- | 12-15 | |
| A | DE 10 2004 040735 A1 (ETH ZUERICH INST FUER LEBENSMI [CH]; KINEMATICA AG LITTAU [CH]; ION BO) 9. März 2006 (2006-03-09) * das ganze Dokument * ----- | 12-15 | |
| A | WO 97/22398 A1 (IXTLAN AG [LI]; MAVAG VERFAHRENSTECH AG [CH]; BRESCIANI GIUSEPPE [CH];) 26. Juni 1997 (1997-06-26) * das ganze Dokument * ----- | 12-15 | RECHERCHIERTE SACHGEBIETE (IPC) |

Der vorliegende Recherchenbericht wurde für alle Patentansprüche erstellt

| Recherchenort | Abschlußdatum der Recherche | Prüfer |
|---|---|---|
| Den Haag | 31. Mai 2011 | Tarallo, Anthony |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
..................................................................................................
& : Mitglied der gleichen Patentfamilie, übereinstimmendes Dokument

EPO FORM 1503 03.82 (P04C03)

Europäisches
Patentamt

European
Patent Office

Office européen
des brevets

**Nummer der Anmeldung**

EP 10 16 7570

---

## GEBÜHRENPFLICHTIGE PATENTANSPRÜCHE

Die vorliegende europäische Patentanmeldung enthielt bei ihrer Einreichung Patentansprüche, für die eine Zahlung fällig war.

☐ Nur ein Teil der Anspruchsgebühren wurde innerhalb der vorgeschriebenen Frist entrichtet. Der vorliegende europäische Recherchenbericht wurde für jene Patentansprüche erstellt, für die keine Zahlung fällig war, sowie für die Patentansprüche, für die Anspruchsgebühren entrichtet wurden, nämlich Patentansprüche:

☐ Keine der Anspruchsgebühren wurde innerhalb der vorgeschriebenen Frist entrichtet. Der vorliegende europäische Recherchenbericht wurde für die Patentansprüche erstellt, für die keine Zahlung fällig war.

---

## MANGELNDE EINHEITLICHKEIT DER ERFINDUNG

Nach Auffassung der Recherchenabteilung entspricht die vorliegende europäische Patentanmeldung nicht den Anforderungen an die Einheitlichkeit der Erfindung und enthält mehrere Erfindungen oder Gruppen von Erfindungen, nämlich:

Siehe Ergänzungsblatt B

☒ Alle weiteren Recherchengebühren wurden innerhalb der gesetzten Frist entrichtet. Der vorliegende europäische Recherchenbericht wurde für alle Patentansprüche erstellt.

☐ Da für alle recherchierbaren Ansprüche die Recherche ohne einen Arbeitsaufwand durchgeführt werden konnte, der eine zusätzliche Recherchengebühr gerechtfertigt hätte, hat die Recherchenabteilung nicht zur Zahlung einer solchen Gebühr aufgefordert.

☐ Nur ein Teil der weiteren Recherchengebühren wurde innerhalb der gesetzten Frist entrichtet. Der vorliegende europäische Recherchenbericht wurde für die Teile der Anmeldung erstellt, die sich auf Erfindungen beziehen, für die Recherchengebühren entrichtet worden sind, nämlich Patentansprüche:

☐ Keine der weiteren Recherchengebühren wurde innerhalb der gesetzten Frist entrichtet. Der vorliegende europäische Recherchenbericht wurde für die Teile der Anmeldung erstellt, die sich auf die zuerst in den Patentansprüchen erwähnte Erfindung beziehen, nämlich Patentansprüche:

☐ Der vorliegende ergänzende europäische Recherchenbericht wurde für die Teile der Anmeldung erstellt, die sich auf die zuerst in den Patentansprüchen erwähnte Erfindung beziehen (Regel 164 (1) EPÜ).

**Europäisches Patentamt**
**European Patent Office**
**Office européen des brevets**

**MANGELNDE EINHEITLICHKEIT
DER ERFINDUNG
ERGÄNZUNGSBLATT B**

**Nummer der Anmeldung**

EP 10 16 7570

Nach Auffassung der Recherchenabteilung entspricht die vorliegende europäische Patentanmeldung nicht den Anforderungen an die Einheitlichkeit der Erfindung und enthält mehrere Erfindungen oder Gruppen von Erfindungen, nämlich:

1. Ansprüche: 1-11

   Verfahren zur Herstellung von Vesikeln.
   ---

2. Ansprüche: 12-15

   Vorrichtung zur Herstellung von Vesikeln.
   ---

**ANHANG ZUM EUROPÄISCHEN RECHERCHENBERICHT**
**ÜBER DIE EUROPÄISCHE PATENTANMELDUNG NR.**

EP 10 16 7570

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

31-05-2011

| Im Recherchenbericht angeführtes Patentdokument | Datum der Veröffentlichung | Mitglied(er) der Patentfamilie | Datum der Veröffentlichung |
|---|---|---|---|
| EP 1920765 A1 | 14-05-2008 | AU 2007316931 A1<br>CA 2668743 A1<br>EP 2094239 A1<br>WO 2008055628 A1<br>JP 2010508368 T<br>US 2010316696 A1 | 15-05-2008<br>15-05-2008<br>02-09-2009<br>15-05-2008<br>18-03-2010<br>16-12-2010 |
| EP 0649867 A1 | 26-04-1995 | DE 69330954 D1 | 22-11-2001 |
| WO 2008009623 A1 | 24-01-2008 | AR 062087 A1<br>AT 470495 T<br>AU 2007276190 A1<br>CA 2655886 A1<br>CL 20892007 A1<br>CN 101489657 A<br>EP 2043769 A1<br>ES 2345292 T3<br>PE 03862008 A1<br>RU 2009105254 A<br>US 2009323459 A1<br>ZA 200901082 A | 15-10-2008<br>15-06-2010<br>24-01-2008<br>24-01-2008<br>11-04-2008<br>22-07-2009<br>08-04-2009<br>20-09-2010<br>23-05-2008<br>27-08-2010<br>31-12-2009<br>28-04-2010 |
| DE 2054968 A1 | 10-05-1972 | CH 535060 A<br>FR 2113608 A5<br>GB 1352629 A<br>IT 940452 B<br>SE 378762 B<br>US 3797662 A | 31-03-1973<br>23-06-1972<br>08-05-1974<br>10-02-1973<br>15-09-1975<br>19-03-1974 |
| FR 2652762 A1 | 12-04-1991 | KEINE | |
| SU 1442258 A | 07-12-1988 | KEINE | |
| SU 606609 A | 13-04-1978 | KEINE | |
| RU 2088870 C1 | 27-08-1997 | KEINE | |
| RU 2000067 C | 07-09-1993 | KEINE | |
| US 5312544 A | 17-05-1994 | KEINE | |
| DE 102004040735 A1 | 09-03-2006 | AT 387255 T<br>EP 1781402 A1<br>WO 2006021375 A1<br>JP 2008510607 T<br>US 2011038901 A1 | 15-03-2008<br>09-05-2007<br>02-03-2006<br>10-04-2008<br>17-02-2011 |

EPO FORM P0461

Für nähere Einzelheiten zu diesem Anhang : siehe Amtsblatt des Europäischen Patentamts, Nr.12/82

# EP 2 402 075 A1

EP 10 16 7570

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

31-05-2011

| Im Recherchenbericht angeführtes Patentdokument | Datum der Veröffentlichung | Mitglied(er) der Patentfamilie | Datum der Veröffentlichung |
|---|---|---|---|
| WO 9722398 A1 | 26-06-1997 | EP 0876186 A1<br>FR 2742352 A1<br>JP 2000501651 T | 11-11-1998<br>20-06-1997<br>15-02-2000 |

Für nähere Einzelheiten zu diesem Anhang : siehe Amtsblatt des Europäischen Patentamts, Nr.12/82

EPO FORM P0461

23

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 10159108 A
- EP 664116 A
- WO 8900846 A
- WO 9800228 A